# FASCICULE DE BREVET EUROPEEN

(11) **EP 2 008 110 B1**
(45) Date de publication et mention de la délivrance du brevet: **22.03.2017**
(21) Numéro de dépôt: 07731293.2
(22) Date de dépôt: 13.04.2007
(51) Int. Cl.: G01N 33/94

(54) **METHODE DE DIAGNOSTIC IN VITRO OU EX VIVO DE DESORDRES PSYCHIATRIQUES ET/OU DE DYSBIOSES INTESTINALES**
VERFAHREN ZUR IN-VITRO- ODER EX-VIVO-DIAGNOSE PSYCHIATRISCHER STÖRUNGEN UND/ODER INTESTINALEN DYSBIOSEN
METHOD FOR DIAGNOSING IN VITRO OR EX VIVO PSYCHIATRIC DISORDERS AND/OR INTESTINAL DYSBIOSES

(30) Priorité: 14.04.2006 FR 0603346
(43) Date de publication de la demande: 31.12.2008
(73) Titulaire: Université Henri Poincaré Nancy 1, 54003 Nancy Cedex (FR)
(72) Inventeur: CHERY, Céline, F-54000 Nancy (FR); LEFEBVRE, Emmanuelle, F-54500 Vandoeuvre (FR); MERTEN, Marc, F-54370 Einville-au-Jard (FR)
(74) Mandataire: Santarelli
(86) Numéro de dépôt international: PCT/FR2007/000626
(87) Numéro de publication internationale: WO 2007/119004

(56) Documents cités:
- US-A- 5 686 311
- US-A- 5 783 680
- MICHAUX R: "SEARCH FOR URINARY CATABOLITES POSSIBLE CHARACTERISTIC OF SCHIZOPHRENIA / RECHERCHE DES CATABOLITES URINAIRES EVENTUELLEMENT CARACTERISTIQUES DE LA SCHIZOPHRENIE" PATHOLOGIE ET BIOLOGIE, vol. 16, no. 15-18, 1968, pages 731-734, XP008072706
- HELEVUO H: "Primary and secondary catecholamine excretion in normal and autistic children" ANTHROPOLOGIAI KOZLEMENYEK, EOTVOS LORAND TUDOMANYEGYETEM, vol. 24, no. 1-2, 1980, pages 105-117, XP008072743
- SHI RUN ZHANG ET AL: "Development of an enzyme-linkied immunosorbent assay with monoclonal antibody for quantification of homovanillic in human urine samples" CLINICAL CHEMISTRY, vol. 44, no. 8 PART 1, 1998, pages 1674-1679, XP002411704
- MROCHEK J E ET AL: "MONITORING PHENYL ALANINE TYROSINE METABOLISM BY HIGH RESOLUTION LIQUID CHROMATOGRAPHY OF URINE" CLINICAL CHEMISTRY, vol. 19, no. 8, 1973, pages 927-936, XP002411702
- MUUSZE R G: "THE LC ANALYSIS OF CATECHOLAMINE METABOLITES IN URINE" CHROMATOGRAPHIC SCIENCE, vol. 20, no. 4, 1982, pages 257-278, XP008072701
- MITCHELL J ET AL: "APPLICATION OF PAIRED ION HIGH PRESSURE LIQUID COLUMN CHROMATOGRAPHY TO THE ANALYSIS OF L DOPA METABOLITES" JOURNAL OF CHROMATOGRAPHY BIOMEDICAL APPLICATIONS, vol. 145, no. 2, 1978, pages 295-301, XP002462016
- DALGLIESH CE ET AL: "A Gas-Liquid-Chromatographic Procedure for Separating a Wide Range of Metabolites Occurring in Urine or Tissue Extracts" THE BIOCHEMICAL JOURNAL, vol. 101, 1966, pages 792-810, XP002462017
- SETA K ET AL: "HIGH PERFORMANCE ANION EXCHANGE CHROMATOGRAPHY OF UV ABSORBING CONSTITUENTS OF HUMAN URINE" JOURNAL OF CHROMATOGRAPHY BIOMEDICAL APPLICATIONS, vol. 181, no. 3-4, 1980, pages 311-318, XP002462018
- YOSHIDA A ET AL: "DETERMINATION OF VANILPYRUVIC ACID IN URINE BY HIGH-SPEED LIQUID CHROMATOGRAPHY" CHEMICAL AND PHARMACEUTICAL BULLETIN, vol. 26, no. 4, 1978, pages 1177-1181, XP008072726
- HICKS J M ET AL: "Abnormal blood constituents in acute renal failure." CLINICA CHIMICA ACTA, vol. 7, 1962, pages 623-633, XP002449193
- SAINI A S: "SEPARATION OF PHENOLIC ACIDS AND INDOLIC ACIDS FROM UNTREATED URINE" JOURNAL OF CHROMATOGRAPHY, vol. 54, no. 3, 1971, pages 442-445, XP002449194
- MASHIGE ET AL: "DEVELOPMENT OF A HIGH-PERFORMANCE LIQUID CHROMATOGRAPHY SYSTEM WITH MULTI-ELECTRODE ELECTROCHEMICAL DETECTORS FOR DETERMINATION OF LEVELS OF NEUROTRANSMITTERS IN CEREBROSPINAL FLUID" RINSHO KAGAKU - JAPANESE JOURNAL OF CLINICAL CHEMISTRY, vol. 22, no. 3, 1993, pages 147-155, XP008072708
- MCDOUGLE C J ET AL: "NEUROCHEMISTRY IN THE PATHOPHYSIOLOGY OF AUTISM" JOURNAL OF CLINICAL PSYCHIATRY, vol. 66, no. SUPPL 10, 2005, pages 9-18, XP008072685
- NARAYAN MEENA ET AL: "Cerebrospinal fluid levels of homovanillic acid and 5-hydroxyindoleacetic acid in autism" BIOLOGICAL PSYCHIATRY, vol. 33, no. 8-9, 1993, pages 630-635, XP002411700
- MINDERAA RUUD B ET AL: "Noradrenergic and adrenergic functioning in autism" BIOLOGICAL PSYCHIATRY, vol. 36, no. 4, 1994, pages 237-241, XP002411701
- ISSACHAR D ET AL: "Screening of organic acids in urine by chemical ionization mass spectrometry" BIOMEDICAL MASS SPECTROMETRY, vol. 6, no. 2, février 1979 (1979-02), pages 47-56, XP002411703
- MARTINEAU J ET AL: "EFFECTS OF VITAMIN B-6 ON AVERAGED EVOKED POTENTIALS IN INFANTILE AUTISM" BIOLOGICAL PSYCHIATRY, vol. 16, no. 7, 1981, pages 627-641, XP008072683
- LELORD G ET AL: "MODIFICATIONS IN URINARY HOMO VANILLIC-ACID AFTER INGESTION OF VITAMIN B-6 FUNCTIONAL STUDY IN AUTISTIC CHILDREN" REVUE NEUROLOGIQUE, vol. 134, no. 12, 1978, pages 797-801, XP008072687
- BARTHELEMY C ET AL: "BIOLOGICAL AND CLINICAL EFFECTS OF ORAL MAGNESIUM AND ASSOCIATED MAGNESIUM VITAMIN B-6 ADMINISTRATION ON CERTAIN DISORDERS OBSERVED IN INFANTILE AUTISM" THERAPIE, vol. 35, no. 5, 1980, pages 627-632, XP008072688
- LIEBICH H M ET AL: "BASIC PROFILES OF ORGANIC ACIDS IN URINE" JOURNAL OF CHROMATOGRAPHY, vol. 525, no. 1, 1990, pages 1-14, XP002411699

## Description

La présente invention concerne un nouveau marqueur biologique pour le diagnostic de désordres psychiatriques et/ou de dysbioses intestinales.

Plus particulièrement, l'invention concerne une méthode de diagnostic, notamment *in vitro* ou *ex vivo* de désordres psychiatriques, notamment d'autisme, et/ou de dysbioses intestinales.

Plus particulièrement, l'invention concerne une méthode de diagnostic, notamment *in vitro* ou *ex vivo* d'autisme, ou de dysbioses intestinales associées à l'autisme.

L'autisme chez l'enfant, est un désordre psychiatrique hétérogène du point de vue des mécanismes de pathogénécité. Cette pathologie, aussi nommée syndrome de Kanner, survient habituellement dans la petite enfance, dans la première, deuxième ou troisième année de l'enfant. Elle se caractérise par un désordre du langage, une incapacité à développer des relations sociales, par des comportements rituels et répétitifs et enfin par des retards mentaux plus ou moins importants.

Il existe de fortes présomptions en faveur d'un terrain génétique de cette maladie. Toutefois, de plus en plus de recherches lient le dysfonctionnement du cerveau à un déséquilibre métabolique dont l'origine serait dans les intestins. Des déséquilibres de la flore intestinale, appelés dysbioses intestinales, sont très fréquemment rencontrés chez les personnes atteintes de troubles psychiatriques.

Le diagnostic de désordres psychiatriques et notamment de l'autisme est habituellement basé sur des observations cliniques attentives de l'individu. Le diagnostic par observation est compliqué par le fait, d'une part, que beaucoup d'autres désordres du système nerveux central peuvent aussi avoir des symptômes très comparables à ceux de l'autisme, et d'autre part, par le fait que ces observations fastidieuses de l'enfant, doivent être répétées et prolongées dans le temps et effectuées par des spécialistes expérimentés en milieu hospitalier.

La demande de brevet EP 0 979 828, concerne une méthode de diagnostic de l'autisme comprenant la détection de peptides de formule pyroGlu-Trp-GlyNH2, Gly-Ser-Glu-Asn et pyroGlu-Glu-Asp-Ser, dans un fluide biologique tel que le sang ou l'urine, par des méthodes appropriées de type HPLC ou immunoprécipitation grâce à des anticorps dirigés contre les peptides précités. Il est donné comme hypothèse dans cette demande, que les taux élevés des métabolites cités ci-dessus chez les patients autistes ont comme origine possible une infection fongique.

La demande de brevet US 5, 686,311 expose une méthode de diagnostic de l'autisme basée sur la détermination d'une série de métabolites qui 1) sont susceptibles d'agir sur le Cycle de Krebs ou 2) sont d'origine bactérienne, tels que l'acide citramalique, l'acide citrique, l'acide tartrique, l'acide furan-2,5-dicarboxylique, l'acide 3-oxo-glutarique, l'acide dihydroxyphénylpropionique, l'acide carboxycitrique, l'acide 5-hydroxyméthyl-2-furoïque, l'acide furancarboxyglycine, l'acide phénylcarboxylique et l'arabinose. Ces composés sont détectés par GC/MS dans des fluides biologiques (urine, sang, salive et liquide cérébrospinal) de patients autistes et leurs taux sont comparés à ceux détectés dans des échantillons issus de patients sains. Cependant, l'utilisation de ces métabolites comme marqueurs biologiques ne semble pas toujours permettre de diagnostiquer des désordres psychiatriques sans avoir recours à l'étude des symptômes cliniques.

Reichelt et al. (Biol. Psychiatry, 1986, 21 (13) 1279-90; Adv. Biochem. Psychopharmacol, 1981, 28, p.627-43 ; Med. Hypothese, 1995, 45 (5), p. 498-502) ont détecté un ensemble de peptides chez les enfants autistes et ont purifié certains de ces peptides présents dans leurs urines. Dans le but d'identifier des patients chez qui le syndrome de l'autisme serait causé par un mécanisme pathogène commun, ces peptides tels que le gluten et la caséine sont recherchés. Les peptides précités semblent n'être détectés que chez certains patients et non pas dans l'ensemble des cas d'autisme.

Des publications concernent l'étude de l'autisme par la mesure des taux d'un métabolite dopaminergique majeur, l'acide homovanilique (HVA), ou d'un métabolite issu de la norépinéphrine, l'acide vanillylmandélique (VMA), dans un fluide biologique.

Ces publications mentionnent que l'autisme semble être lié à un désordre neurobiologique mais présentent des résultats contradictoires.

A titre d'exemple, on peut citer celle de Robert T. Schultz et al. (Charney, D.S. and Nestler, E.J. The neurobiology of Mental Illness (2nd edition), Oxford University Press*),* dans laquelle est indiqué que le niveau de HVA dans le liquide cérébrospinal de personnes autistes est soit augmenté, soit diminué, soit identique à celui de personnes saines ; de même certaines études du niveau urinaire de HVA rapportent que les niveaux sont similaires chez les sujets autistes et les sujets sains.

Dans la publication de Christopher J. McDougle (J Clin Psychiatry 2005 ;66 (suppl 10)), il est également décrit que le taux urinaire de HVA d'individus autistes ne diffère pas de celui des individus sains.

De même, dans la publication de Minderaa RB (Noradrenergic and adrenergic functioning in autism, Biol Psychiatry. 1994 Aug 15; 36(4) :237-41. PMID: 79866888), le taux urinaire de l'acide vanillylmandélique chez des individus sains et autistes est mesuré (tableau 5 de la publication). Il est montré que les individus autistes ont un taux urinaire d'acide vanillylmandélique identique à celui des contrôles.

A ce jour, les méthodes de l'art antérieur ne fournissent pas encore de solution satisfaisante au problème du diagnostic rapide et sûr de l'autisme au moyen de marqueurs biologiques, sachant que chacun d'entre eux est intrinsèquement insuffisant pour discriminer une condition physiologique d'une situation pathologique.

L'un des buts de l'invention est de fournir un nouveau marqueur biologique sensible, fiable et d'utilisation rapide, pour le diagnostic de désordres psychiatriques, notamment l'autisme et/ou de dysbioses intestinales.

L'un des buts de l'invention est également de fournir un kit de diagnostic de désordres psychiatriques, notamment l'autisme et/ou de dysbioses intestinales.

L'un des buts de l'invention est de fournir des anticorps monoclonaux et polyclonaux et des hybridomes susceptibles de produire ces anticorps, lesquels peuvent être utilisés dans le kit de diagnostic ou comme antagonistes pour le traitement de désordres psychiatriques et/ou de dysbioses intestinales.

La présente invention concerne l'utilisation de l'un au moins des composés de formule (I) suivante : 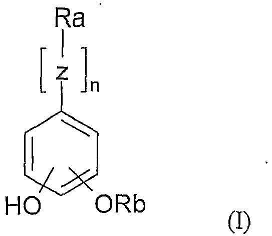 dans laquelle :
* n = 0 ou 1,
* Ra représente -COOH,
* Rb représente un groupe alkyle de 1 à 3 atomes de carbone, de préférence un groupe -CH₃,
* Z représente -CHOH-CH₂-, -CH₂-CH₂-, -CO-NH-CH₂-
* lesdits composés étant le cas échéant sous forme de sels ou lorsqu'il y a présence d'au moins un carbone asymétrique, sous forme d'isomères seuls ou en mélange racémique,
* lesdits composés étant différents de l'acide vanillyllactique et de l'acide homovanillique,
comme marqueur physiologique pour la mise en oeuvre d'une méthode de diagnostic *in vitro* ou *ex vivo* de désordres psychiatriques appartenant au groupe constitué de l'autisme, des retards de développement et de l'autisme lié à la dysbiose intestinale.

Dans la présente invention, il a été trouvé de façon surprenante que chez les patients atteints de désordres psychiatriques, le pic de valeur de l'un au moins des composés définis ci-dessus, est significativement supérieur à la valeur obtenue pour un patient sain.

Il a également été trouvé de façon inattendue, que la présence de l'un au moins des composés définis ci-dessus dans un échantillon biologique d'un malade permet avantageusement de diagnostiquer l'autisme, ou des dysbioses intestinales associées à l'autisme.

En d'autres termes, de façon avantageuse, les composés utilisables selon l'invention étant spécifiques du diagnostic de désordres psychiatriques (éventuellement associés à des dysbioses), ils ne permettent pas le diagnostic de dysbioses non associées à des désordres psychiatriques,

On entend par « marqueur physiologique » un marqueur biochimique permettant la mise en oeuvre d'une méthode de diagnostic in vitro ou ex vivo.

On entend,par sels, des sels pharmacologiquement acceptables tels que les sels d'ammonium, sodium, potassium, calcium, magnésium.

Le terme « désordres psychiatriques », tel qu'utilisé dans la présente invention, se réfère à des désordres psychiatriques ou neurologiques. Les désordres neurologiques concernent les troubles du système nerveux. Les désordres psychiatriques concernent les classifiés dans Diagnostic and Statistical Manual of Mental Disorders, Fourth Edition, Revised : DSM-IV-TR, Washington, DC : American Psychiatric Association ; 2000.

L'expression « dysbiose intestinale » définie un déséquilibre de la flore intestinale couramment observé chez des individus atteints de désordres psychiatriques de type autisme.

Selon un mode de réalisation avantageux, la présente invention concerne l'utilisation telle que définie ci-dessus, de l'un au moins des composés de formule (I') suivante : 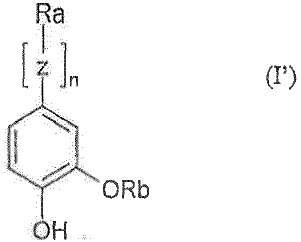 dans laquelle :
n = 0 ou 1,
* Ra représente COOH,
* Rb représente un groupe alkyle de 1 à 3 atomes de carbone, de préférence un groupe -CH₃,
* Z représente -CHOH-CH₂-, -CH₂-CH₂, -CO-NH-CH₂-,
* lesdits composés étant le cas échéant sous forme de sels ou lorsqu'il y a présence d'au moins un carbone asymétrique, sous forme d'isomères seuls ou en mélange racémique,
* lesdits composés étant différents de vanillyllactique et de l'acide homovanillique,
comme marqueur physiologique pour la mise en oeuvre d'une méthode de diagnostic *in vitro* ou *ex vivo* de désordres psychiatriques appartenant au groupe constitué de l'autisme, des retards de développement et de l'autisme lié à la dysbiose intestinale.

Selon un autre mode de réalisation avantageux, la présente invention concerne l'utilisation telle que définie ci-dessus, de l'un au moins des composés de formule (II) suivante: 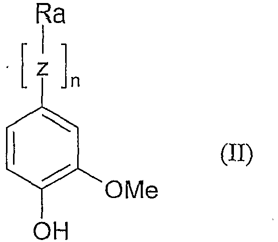 dans laquelle n, Ra, et Z sont tels que définis ci-dessus.

Selon un mode de réalisation avantageux, la présente invention concerne l'utilisation telle que définie ci-dessus, de l'un au moins des composés de formule (I) ou (II), dans laquelle :
* n = 1,
* Ra représente -COOH,
* Rb représente -CH₃-,
* Z représente -CHOH_CH₂-,
* lesdits composés étant différents de l'acide vanillyllactique.

Selon un mode de réalisation avantageux de l'invention, le composé de formule (II) est choisi parmi l'un des composés suivants :

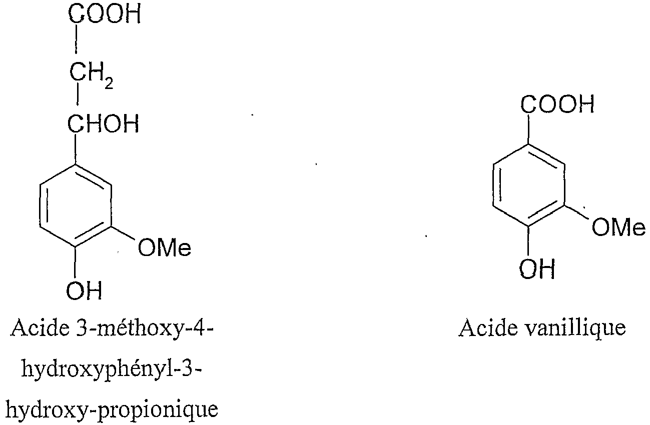

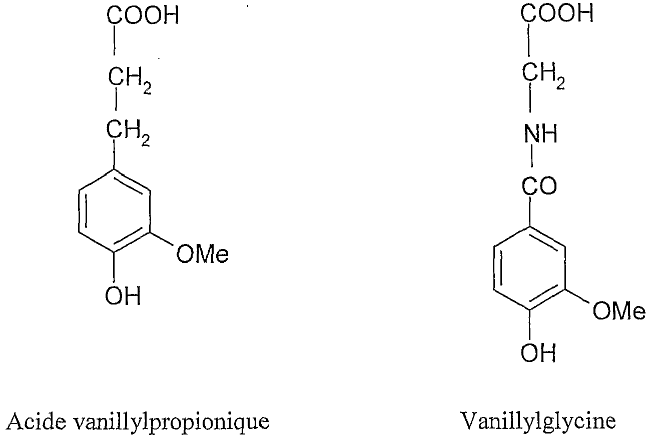

Selon un autre mode de réalisation avantageux de l'invention, le composé de formule (II) utilisé est l'acide 3-méthoxy-4-hydroxyphényl-3-hydroxy-propionique et répond à la formule suivante :

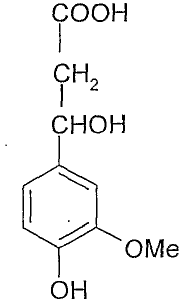

Le composé tel que défini ci-dessus est l'acide 3-méthoxy-4-hydroxyphényl-3-hydroxy-propionique, nommé aussi acide vanillyl-3OH-propionique ou vanillylhydracrylique. Ce composé est un produit intermédiaire d'une des voies de dégradation bactérienne de l'acide férulique (provenant des polyphénols alimentaires).

Dans la présente invention, il a été trouvé de façon surprenante que chez les patients atteints de désordres psychiatriques, le pic de valeur de l'acide 3-méthoxy-4-hydroxyphényl-3-hydroxy-propionique est significativement supérieur à la valeur obtenue pour un patient sain. Les niveaux significatifs dudit composé, mis en évidence par des méthodes appropriées, notamment la GC/MS, chez tout les patients analysés, permettent de considérer ledit composé comme un marqueur sensible et fiable pour le diagnostic de l'autisme et autres désordres psychiatriques.

Selon un autre mode de réalisation avantageux de l'invention, le composé de formule (II) utilisé est l'acide vanillique et répond à la formule suivante :

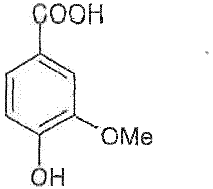

Dans la présente invention, il a été trouvé de façon surprenante que chez les patients atteints de désordres psychiatriques, le pic de valeur de l'acide vanillique est significativement supérieur à la valeur obtenue pour un patient sain.

Les composés peuvent être utilisés seuls ou en mélange, par exemple l'acide Vanillylpropionique (VPA) en mélange avec la Vanillylglycine (VG) ; ou l'acide Vanillylpropionique (VPA) en mélange avec l'acide Vanillique (VA) ; ou l'acide Vanillylpropionique (VPA) en mélange avec la Vanillylglycine (VG) et l'acide Vanillique (VA) ; ou encore l'acide Vanillique (VA) en mélange avec la Vanillylglycine (VG).

Les désordres psychiatriques impliqués dans l'invention appartiennent au groupe constitué de l'autisme, des retards de développement et de l'autisme lié à la dysbiose intestinale.

Selon un autre mode de réalisation avantageux, le désordre psychiatrique impliqué dans l'invention, est l'autisme.

De façon avantageuse, le désordre psychiatrique impliqué dans l'invention est l'autisme lié à la dysbiose intestinale.

La présente invention concerne également, une méthode de diagnostic et/ou de pronostic, notamment in vitro ou ex vivo, de désordres psychiatriques appartenant au groupe constitué de l'autisme, des retards de développement et de l'autisme lié à la dysbiose intestinale, caractérisée en ce qu'elle comprend une étape de détermination d'une variation de la quantité de l'un au moins des composés définis ci-dessus, présente dans un échantillon biologique prélevé sur un patient, par rapport à la quantité de ce(s) même(s) composé(s) présente dans un échantillon biologique prélevé chez un individu

Le diagnostic est positif si la variation de la quantité pour au moins l'un des composés, par exemple dans un échantillon urinaire et notamment pour l'acide 3-méthoxy-4-hydroxyphényl-3-hydroxy-propionique, est supérieure à 20 microgrammes par milligramme de créatinine, plus préférentiellement de 20 à 180 microgrammes par milligramme de créatinine.

Selon un mode de réalisation avantageux, la présente invention concerne une méthode telle que définie ci-dessus, dans laquelle la détermination par une méthode appropriée, d'une augmentation de la quantité de l'un au moins des composés définis ci-dessus, présente dans un échantillon biologique prélevé sur un patient, par rapport à la quantité de ce(s) même(s) composé(s) présente dans un échantillon biologique prélevé chez un individu sain, correspond au diagnostic de de l'autisme, et/ou de l'autisme lié à la dysbiose intestinale.

Selon un autre mode de réalisation avantageux, la présente invention concerne la méthode telle que définie ci-dessus, dans laquelle la quantité de l'un au moins des composés définis ci-dessus, présente dans un échantillon biologique prélevé sur un patient, et la quantité de ce(s) même(s) composé(s) présente dans un échantillon biologique prélevé chez un individu sain, est mesurée par chromatographie, par spectrophotométrie, par une méthode immunologique, notamment de test ELISA, ou immuno-néphélémétrique et préférentiellement par chromatographie en phase gazeuse couplée à une spectrométrie de masse (GC/MS).

Ces procédés sont connus et utilisés par l'homme de l'art et sont appliqués avec succès pour l'analyse d'échantillons biologiques en routine (sérum, urine, sang, etc.) afin de doser un grand nombre d'analytes, tels que les protéines, les peptides, les stéroïdes naturels ou les drogues. Ces procédés sont référencés dans l'ouvrage de P. Tijssen « Practice and Theory of Enzyme Immunoassays », Elservier, Amsterdam 1985. Toutefois, le principal avantage de la technique GC/MS est sa simplicité de manipulation, son faible temps d'analyse, et avant tout, sa grande. spécificité et sensibilité de détection qui permet une caractérisation plus fine dudit composé dans des échantillons biologiques, en comparaison aux autres méthodes connues de l'homme de l'art.

Dans la présente invention, l'analyse et la quantification de l'acide 3-méthoxy-4-hydroxyphényl-3-hydroxy-propionique par GC/MS peut être effectuée sur la base de fragments ioniques caractéristiques de rapport m/z de 297 et 428.

Selon un mode de réalisation avantageux de l'invention, dans la méthode telle que définie ci-dessus, la détermination d'une augmentation de la quantité dudit composé de formule : 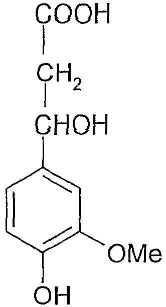 présente dans un échantillon biologique prélevé sur un patient, par rapport à la quantité de ce même composé présente dans un échantillon biologique prélevé chez un individu sain, correspond au diagnostic de l'autisme, des retards de développement et de l'autisme lié à la dysbiose intestinale.

Selon un mode de réalisation avantageux, l'échantillon biologique impliqué dans l'invention est choisi parmi l'urine, le sang, la salive, le liquide cérébrospinal ou les selles.

Selon un autre mode de réalisation avantageux, la présente invention concerne la méthode telle que définie ci-dessus, dans laquelle la quantité de l'un au moins des composés définis ci-dessus, notamment la quantité du composé acide 3-méthoxy-4-hydroxyphényl-3-hydroxy-propionique, présente dans un échantillon d'urine prélevé sur un patient, est supérieure à environ 20 micro grammes par milligramme de créatinine et notamment de 20 à 180 microgrammes par milligramme de créatinine.

La présente divulgation concerne des anticorps monoclonaux ou polyclonaux dirigés contre l'un au moins des composés définis ci-dessus, en particulier dirigés contre l'acide 3-méthoxy-4-hydroxyphényl-3-hydroxy-propionique.

Les anticorps monoclonaux peuvent être obtenus par immunisation de souris avec l'un des composés définis ci-dessus, par exemple l'acide 3-méthoxy-4-hydroxyphényl-3-hydroxy-propionique, couplée à une protéine ou un peptide de couplage selon les techniques classiques connues de l'homme de l'art. Les immunocytes prélevés après immunisation sont fusionnés avec un myélome murin déficient pour l'hypoxanthine guanine phosphoribosyl transférase. Un milieu de sélection permet la survie des hybridomes, et la sélection de l'hybridome secrétant l'anticorps spécifique anti- acide 3-méthoxy-4-hydroxyphényl-3-hydroxy-propionique est effectuée par des techniques immunologiques classiques.

La présente divulgation concerne également, le kit de diagnostic de désordres psychiatriques et/ou de dysbioses intestinales comprenant :
- un ou plusieurs réactifs, notamment anticorps dirigé contre l'un au moins des composés définis ci-dessus, susceptibles de détecter la présence dans un échantillon biologique, de l'un au moins des composés définis ci-dessus,
- éventuellement, un échantillon témoin contenant l'un au moins des composés en quantité prédéterminée indicative du diagnostic positif de désordres psychiatriques et/ou de dysbioses intestinales,
- éventuellement, un échantillon témoin contenant l'un au moins des composés en quantité prédéterminée indicative du diagnostic négatif de désordres psychiatriques et/ou de dysbioses intestinales.

La technique utilisée pour la réalisation du kit est de type ELISA compétitive. Elle est basée sur la compétition entre l'un des composés définis ci-dessus, par exemple l'acide 3-méthoxy-4-hydroxyphényl-3-hydroxy-propionique, conjugué à de la péroxydase ou à un fluorochrome ou à un conjugué radiomarqué et l'acide 3-méthoxy-4-hydroxyphényl-3-hydroxy-propionique présent dans l'échantillon et ce pour un nombre limité de sites de fixation (anti-corps absorbés). La quantité de conjugué fixé est détectée par comptage de la radioactivité ou par fluorimétrie ou par l'addition d'un substrat chromogénique ou luminescent suivie d'une détection spectrométrique. La quantification est effectuée par comparaison avec une gamme standard qui est présente dans le kit. L'intensité de la lecture est inversement proportionnelle à la quantité d'acide 3-méthoxy-4-hydroxyphényl-3-hydroxy-propionique présente dans le standard ou l'échantillon.

La présente divulgation concerne des hybridomes susceptibles de produire des anticorps monoclonaux tels que définis ci-dessus, en particulier des anticorps monoclonaux dirigés contre l'acide 3-méthoxy-4-hydroxyphényl-3-hydroxy-propionique.

La présente divulgation concerne l'utilisation d'antagonistes de l'un au moins des composés définis ci-dessus, pour le traitement de désordres psychiatriques et/ou de dysbioses intestinales.

De façon avantageuse, les antagonistes sont choisis parmi les anticorps polyclonaux ou monoclonaux dirigés contre les composés décrits ci-dessus.

La présente invention concerne également, l'utilisation d'anticorps monoclonaux ou polyclonaux dirigés contre l'un au moins des composés définis ci-dessus, notamment dirigés contre l'acide 3-méthoxy-4-hydroxyphényl-3-hydroxy-propionique, en tant que réactifs susceptibles de détecter la présence de l'un au moins desdits composés dans un échantillon biologique, comme marqueur physiologique pour la mise en oeuvre d'une méthode de diagnostic *in vitro* ou *ex vivo* de désordres psychiatriques appartenant au groupe constitué de l'autisme, des retards de développement et de l'autisme lié à la dysbiose intestinale.

### DESCRIPTION DES FIGURES

**Figure 1** **:**

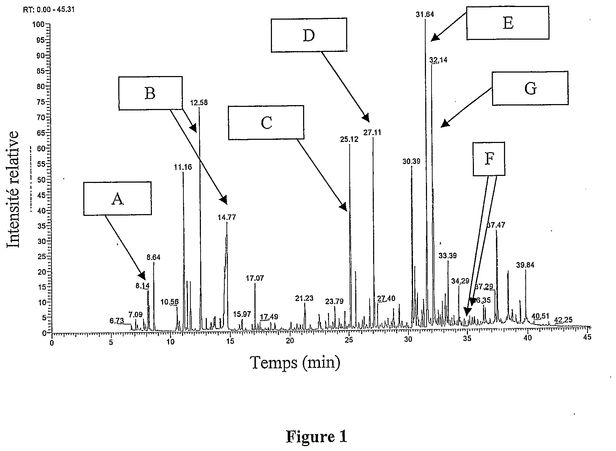

   La figure 1 correspond au chromatogramme obtenu par chromatographie en phase gazeuse couplée à la spectrométrie de masse (GC/MS) après l'analyse d'un échantillon d'urine d'un contrôle sain. Cette figure illustre les concentrations des divers composés dérivés triméthylsilysés (TMS) où les pics sont identifiés tels que : A, acide lactique ; B, urée ; C, étalon interne ; D, acide 2-oxoglutarique ; E, acide hippurique ; F, acide 3-méthoxy-4-hydroxyphényl-3-hydroxy-propionique ; G, acide citrique.
**Figure 2** **:**

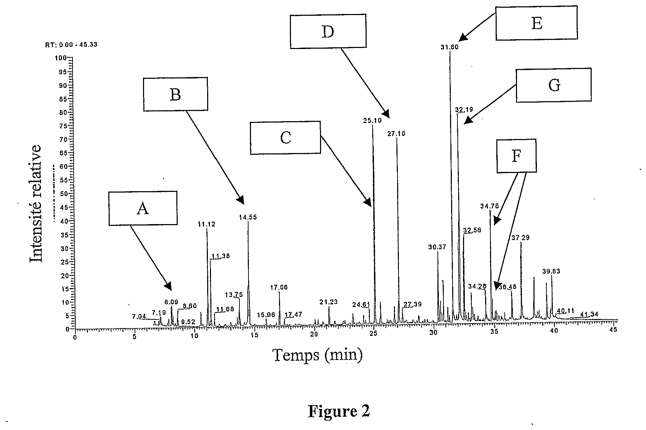

   La figure 2 correspond au chromatogramme obtenu par chromatographie en phase gazeuse couplée à la spectrométrie de masse (GC/MS) après l'analyse d'un échantillon d'urine d'un enfant autiste. Cette figure illustre les concentrations des divers composés dérivés triméthylsilysés (TMS) où les pics sont identifiés tels que : A, acide lactique ; B, urée ; C, étalon interne ; D, acide 2-oxoglutarique ; E, acide hippurique ; F, acide 3-méthoxy-4-hydroxyphényl-3-hydroxy-propionique ; G, acide citrique.
**Figure 3** **:**

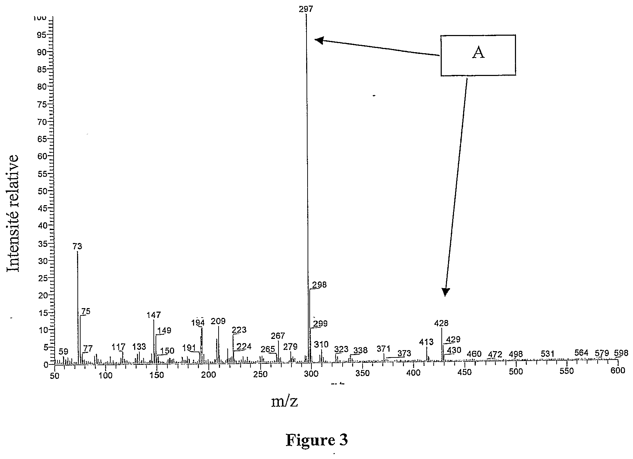

   La figure 3 correspond au chromatogramme obtenu par chromatographie en phase gazeuse couplée à la spectrométrie de masse (GC/MS) permettant de caractériser le spectre de masse du composé identifié comme le dérivé TMS de l'acide 3-méthoxy-4-hydroxyphényl-3-hydroxy-propionique retrouvé chez un patient autiste. L'axe, des abscisses correspond à la masse en Dalton des fragments ioniques. Les fragments ioniques 297 et 428 permettent d'identifier le composé A, acide 3-méthoxy-4-hydroxyphényl-3-hydroxy-propionique.
**Figure 4** **:**

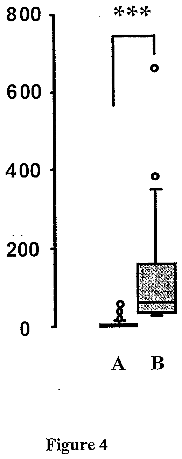

   La figure 4 correspond à l'histogramme comparatif relatif à la quantité du dérivé TMS de l'acide 3-méthoxy-4-hydroxyphényl-3-hydroxy-propionique respectivement entre 27 contrôles sains (A) et 27 patients autistes (B). L'axe des abscisses correspond à la quantité de l'acide 3-méthoxy-4-hydroxyphényl-3-hydroxy-propionique en µg/mg de créatinine. Les valeurs des médianes sont représentées, ainsi que leurs pourcentiles à 95%. La quantité dudit composé chez les patients autistes est supérieure à 20 µg/mg de créatinine et chez les contrôles sains est comprise entre 0 et 15 µg/mg de créatinine. Par ailleurs, des données issues d'analyses chromatographiques démontrent que le rapport de surface de la quantité dudit composé chez les patients autistes est supérieur à 0,1 et chez les contrôles sains est compris entre 0 et 0,07 (résultats non montrés). Le test de Mann et Whitney met en évidence une différence significative avec un Ucal de 3,2.
**Figure 5** **:**

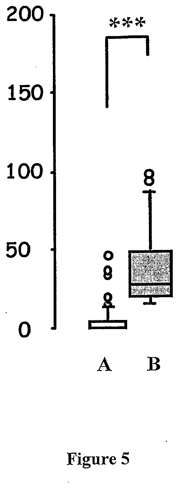

   La figure 5 correspond à l'histogramme comparatif relatif à la quantité du dérivé TMS de l'acide 3-méthoxy-4-hydroxyphényl-3-hydroxy-propionique entre 62 contrôles sains (A) et 45 patients souffrant de pathologies psychiatriques caractérisées par un retard sévère du développement et non autistiques (B). L'axe des abscisses correspond à la quantité de l'acide 3-méthoxy-4-hydroxyphényl-3-hydroxy-propionique en µg/mg de créatinine. Les valeurs des médianes sont représentées ainsi que leurs pourcentiles à 95%. La quantité dudit composé chez les patients atteints de diverses neuropathies non autistiques est supérieure à 20 µg/mg de créatinine et chez les contrôles sains est comprise entre 0 et 15 µg/mg de créatinine. Par ailleurs, des données issues d'analyses chromatographiques démontrent que le rapport de surface entre les taux dudit composé de patients atteints de diverses neuropathies non autistiques et de contrôles sains est compris entre 0 et 0,07 pour les contrôles sains et supérieur à 0,1 pour les patients du groupe (B) (résultats non montrés). Le test de Mann et Whitney met en évidence une différence significative avec un Ucal de 4,74.
**Figure 6** **:**

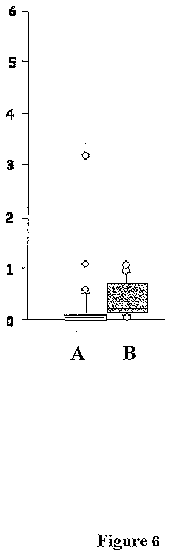

   La figure 6 présente l'histogramme relatif à la quantité du dérivé TMS de la vanillylglycine (VG) respectivement entre 27 contrôles sains (A) et 27 patients autistes (B). L'axe des abscisses correspond au rapport de surface de la quantité de vanillylglycine. Les valeurs des médianes sont représentées, ainsi que leurs pourcentiles à 95%. La valeur de la médiane chez les patients autistes (B) est 0,2 et la valeur de la médiane chez les contrôles sains (A) est 0,04. Pour la vanillylglycine (VG), le rapport de surface de la quantité dudit composé chez les patients autistes est compris entre 0,12 et 0,72 et chez les contrôles sains est compris entre 0 et 0,1. Le test de Mann et Whitney met en évidence une différence significative avec un Ucal de 3,6.
   L'acide vanillique (VA) et l'acide vanillylpropionique (VPA) ont également été testés et ont montré un rapport de surface supérieurs chez les patients autistes à celui des patients sains.
**Figure 7** **:**

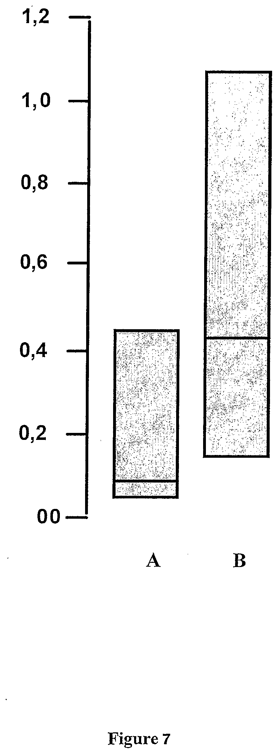

   La figure 7 présente l'histogramme relatif à la quantité du dérivé TMS de l'acide vanillique (VA) respectivement de 38 sujets témoins (A) et de 40 patients autistes (B). L'axe des abscisses correspond au rapport de surface de la quantité d'acide vanillique.
   Les valeurs des médianes sont représentées ainsi que leur pourcentiles à 95%. La valeur de la médiane chez les patients autistes (B) est 0,44 et la valeur de la médiane chez les contrôles sains (A) est 0,124. Par ailleurs, des données issues d'analyses chromatographiques démontrent que le rapport de surface, par rapport à l'étalon interne, de la quantité dudit composé chez les patients autistes est compris entre 0,16 et 1,07 et est compris entre 0,07 et 0,45 chez les contrôles sains. Le test de Mann et Whitney met en évidence une différence significative avec un Ucal de 3,63.
**Figure 8** **:**

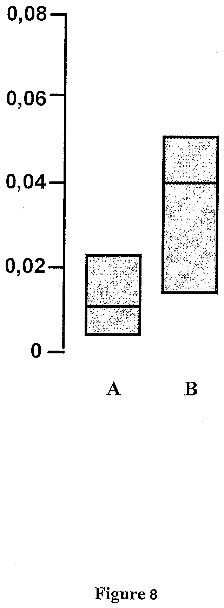

   La figure 8 présente l'histogramme relatif à la quantité du dérivé TMS de l'acide vanillylpropionique (VPA) respectivement de 38 sujets témoins (A) et de 40 patients autistes (B). L'axe des abscisses correspond au rapport de surface de la quantité d'acide vanillylpropionique. Les valeurs des médianes sont représentées ainsi que leur pourcentiles à 95%. La valeur de la médiane chez les patients autistes (B) est 0,04 et la valeur de la médiane chez les contrôles sains (A) est 0,011. Les données issues d'analyses chromatographiques démontrent que le rapport de surface, par rapport à l'étalon interne, de la quantité dudit composé chez les patients autistes est compris entre 0,014 et 0,053 et est compris entre 0,003 et 0,022 chez les contrôles sains. Le test de Mann et Whitney met en évidence une différence significative avec un Ucal de 4,47.
**Figure 9** **:**

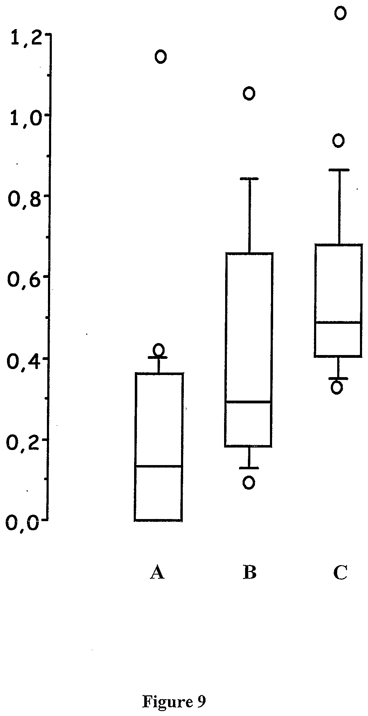

   La figure 9 correspond à l'histogramme comparatif relatif à la quantité du dérivé TMS de l'acide dihydroxyphénylpropionique entre 27 contrôles sains (A), 27 patients autistes (B) et 18 patients non autistes souffrant de dysbioses intestinales (C). Les valeurs des médianes sont représentées ainsi que leurs pourcentiles à 95%. Le rapport de surface de la quantité dudit composé chez les patients non autistes souffrant de dysbioses intestinales est supérieur à celui des patients autistes.

### PARTIE EXPERIMENTALE

### Exemple 1 : Fréquence et quantité de l'acide 3-méthoxy-4-hydroxyphényl-3-hydroxy-propionique chez les patients autistes.

Une analyse GC/MS de l'urine de 27 patients autistes permet d'identifier un pic important ayant les mêmes caractéristiques (temps de rétention et spectre de masse) que l'acide 3-méthoxy-4-hydroxyphényl-3-hydroxy-propionique. Ce pic a été quantifié par intégration de l'aire comprise sous le pic des fragments ioniques spécifiques de rapport m/z de 297 et 428. Une analyse réalisée sur les urines de patients montre que la fréquence des cas ou le composé décrit dans l'invention est détecté dans l'urine est statistiquement différente chez les patients autistes comparée à 27 patients sains. L'étalon interne utilisé est l'acide orthohydroxyphenylacétique dont la quantification est basée sur les ions de fragmentation 296, 281, 252 et 164.

Les résultats sont reportés dans les figures 1 à 4.

### Exemple 2 : Fréquence et quantité de l'acide 3-méthoxy-4-hydroxyphényl-3-hydroxy-propionique chez les patients atteints d'autres désordres psychiatriques.

Une analyse GC/MS de l'urine de 45 patients atteints d'autres désordres psychiatriques permet d'identifier un pic important ayant les mêmes caractéristiques (temps de rétention et spectre de masse) que l'acide 3-méthoxy-4-hydroxyphényl-3-hydroxy-propionique. Ce pic a été quantifié par intégration de l'aire comprise sous le pic des fragments ioniques spécifiques de rapport m/z de 297 et 428. Une analyse réalisée sur les urines de plusieurs patients montre que la fréquence des cas ou le composé décrit dans l'invention est détecté dans l'urine est statistiquement différente chez les patients non autistes mais ayant des désordres psychiatriques et/ou psychomoteurs comparée à 62 patients sains.
L'étalon interne utilisé est l'acide orthohydroxyphenylacétique dont la quantification est basée sur les ions de fragmentation 296, 281, 252 et 164.

Les résultats sont reportés dans la figure 5.

### Exemple 3 : Fréquence et quantité de la vanillylglycine (VG) chez les patients autistes.

Le protocole utilisé est le même que celui de l'exemple 1.

Il a permis d'identifier un pic important ayant les mêmes caractéristiques (temps de rétention et spectre de masse) que la vanillylglycine. Ce pic a été quantifié par intégration de l'aire comprise sous le pic des fragments ioniques spécifiques de rapport m/z de 369, 354 et 223.

Le résultat est reporté dans la figure 6.

### Exemple 4 : Fréquence et quantité de l'acide vanillique (VA) chez les patients autistes.

Le protocole utilisé est le même que celui de l'exemple 1.

Il a permis d'identifier un pic important ayant les mêmes caractéristiques (temps de rétention et spectre de masse) que l'acide vanillique (VA). Ce pic a été quantifié par intégration de l'aire comprise sous le pic des fragments ioniques spécifiques de rapport m/z de 312, 297 et 267.

Le résultat est reporté dans la figure 7.

### Exemple 5 : Fréquence et quantité de l'acide vanillylpropionique (VPA) chez les patients autistes.

Le protocole utilisé est le même que celui de l'exemple 1.

Il a permis d'identifier un pic important ayant les mêmes caractéristiques (temps de rétention et spectre de masse) que l'acide vanillylpropionique (VPA). Ce pic a été quantifié par intégration de l'aire comprise sous le pic des fragments ioniques spécifiques de rapport m/z de 340, 310, et 325.

Le résultat est reporté dans la figure 8.

### Exemple 6 : Fréquence et quantité de l'acide dihydroxyphénylpropionique chez les patients autistes et les patients non autistes souffrant de dysbioses intestinales.

Le protocole utilisé est le même que celui de l'exemple 1, comprenant 18 patients atteints de dysbiose, 27 patients autistes et 27 patients sains.

Il a permis d'identifier un pic important ayant les mêmes caractéristiques (temps de rétention et spectre de masse) que l'acide dihydroxyphénylpropionique. Ce pic a été quantifié par intégration de l'aire comprise sous le pic des fragments ioniques spécifiques de rapport m/z de 398, 267 et 179.

Les résultats de la figure 9, montrent que l'acide dihydroxyphénylpropionique permet le diagnostic de dysbioses non associées à des désordres psychiatriques. En d'autres termes, il n'apparaît pas être un marqueur biologique spécifique du diagnostic de désordres psychiatriques.

## Revendications

1. Utilisation de l'un au moins des composés de formule (I) suivante : 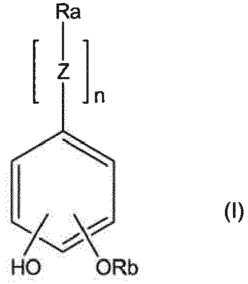 dans laquelle :
*n= 0 ou 1
* Ra représente COOH
* Rb représente un groupe alkyle de 1 à 3 atomes de carbone, de préférence un groupe -CH₃,
* Z représente -CHOH-CH₂-, -CH₂-CH₂-, -CO-NH-CH₂-,
* lesdits composés étant le cas échéant sous forme de sels ou lorsqu'il y a présence d'au moins un carbone asymétrique, sous forme d'isomères seuls ou en mélange racémique,
* lesdits composés étant différents de l'acide vanillyllactique et de l'acide homovanillique,
comme marqueur physiologique pour la mise en oeuvre d'une méthode de diagnostic *in vitro* ou *ex vivo* de désordres psychiatriques appartenant au groupe constitué de l'autisme, des retards de développement et de l'autisme lié à la dysbiose intestinale.

2. Utilisation selon la revendication 1, de l'un au moins des composés de formule (I') suivante : 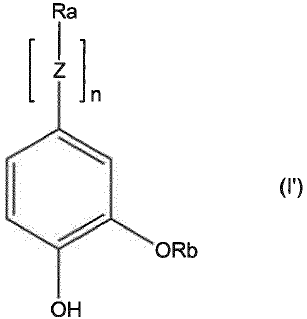 dans laquelle :
* n= 0 ou 1
* Ra représente COOH
* Rb représente un groupe alkyle de 1 à 3 atomes de carbone, de préférence un groupe -CH₃,
* Z représente -CHOH-CH₂-,-CH₂-CH₂-, -CO-NH-CH₂-,
* lesdits composés étant le cas échéant sous forme de sels ou lorsqu'il y a présence d'au moins un carbone asymétrique, sous forme d'isomères seuls ou en mélange racémique,
* lesdits composés étant différents de l'acide vanillyllactique et de l'acide homovanillique,
comme marqueur physiologique pour la mise en oeuvre d'une méthode de diagnostic *in vitro* ou *ex vivo* de désordres psychiatriques appartenant au groupe constitué de l'autisme, des retards de développement et de l'autisme lié à la dysbiose intestinale.

3. Utilisation selon l'une des revendications 1 ou 2, de l'un au moins des composés de formule (II) suivante : 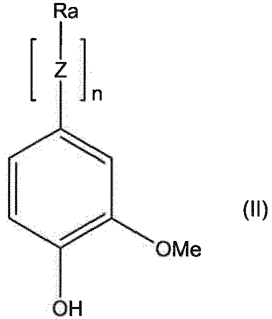 dans laquelle n, Ra, et Z sont tels que définis dans les revendications 1 à 2.

4. Utilisation selon l'une quelconque des revendications 1 à 3, de l'un au moins des composés de formule (I) ou (II) dans laquelle :
* n = 1,
* Ra représente -COOH,
* Rb représente -CH₃-,
* Z représente -CHOH-CH₂-,
* lesdits composés étant différents de l'acide vanillyllactique.

5. Utilisation selon la revendication 3, **caractérisée en ce que** le composé de formule (II) est choisi parmi l'un des composés suivants :

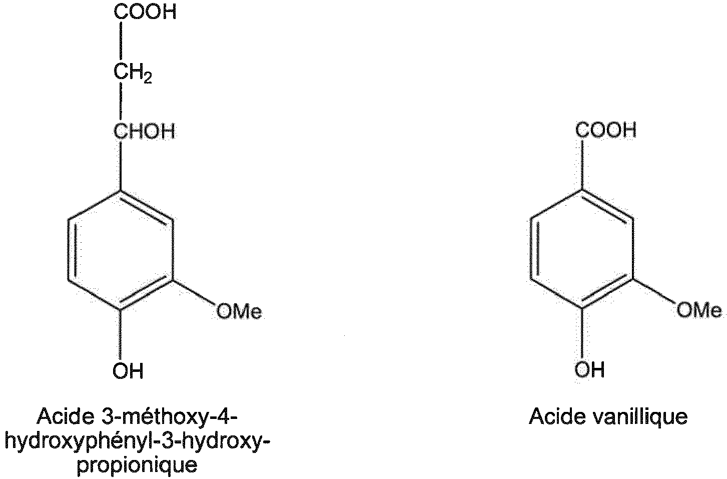

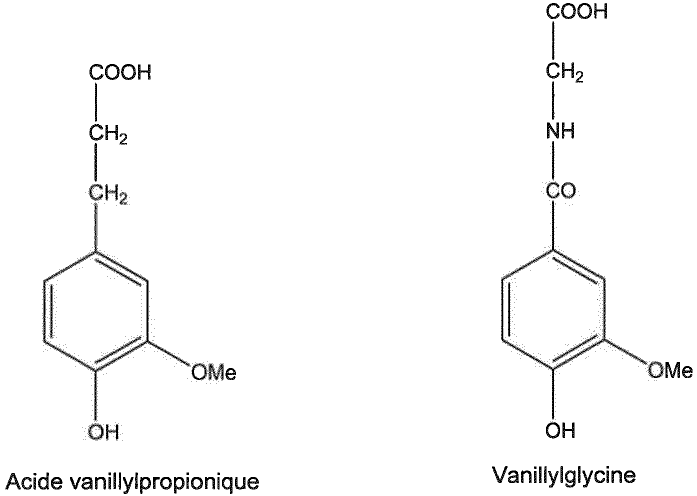

6. Utilisation selon la revendication 3, **caractérisée en ce que** ledit composé répond à la formule suivante :

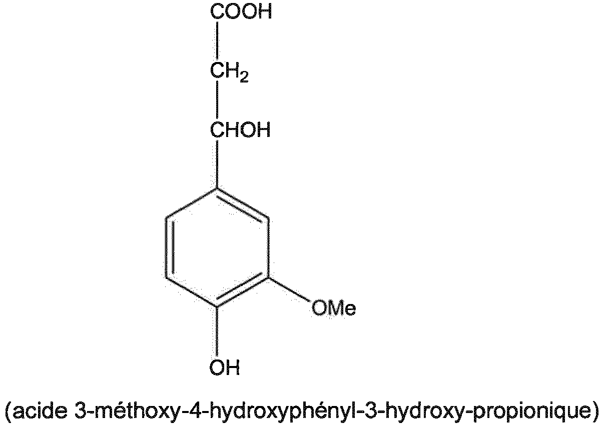

7. Utilisation selon la revendication 3, **caractérisée en ce que** ledit composé répond à la formule suivante :

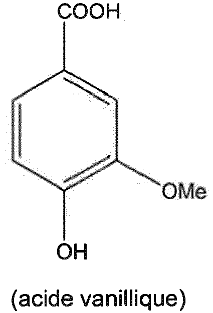

8. Méthode de diagnostic *in vitro* ou *ex vivo* de désordres psychiatriques appartenant au groupe constitué de l'autisme, des retards de développement et de l'autisme lié à la dysbiose intestinale, **caractérisée en ce qu'**elle comprend une étape de détermination d'une variation de la quantité de l'un au moins des composés définis dans l'une des revendications 1 à 7, présente dans un échantillon biologique prélevé sur un patient, par rapport à la quantité de ce(s) même(s) composé(s) présente dans un échantillon biologique prélevé chez un individu sain.

9. Méthode selon la revendication 8, **caractérisée en ce que** la détermination par une méthode appropriée, d'une augmentation de la quantité de l'un au moins des composés définis dans l'une des revendications 1 à 7, présente dans un échantillon biologique prélevé sur un patient, par rapport à la quantité de ce(s) même(s) composé(s) présente dans un échantillon biologique prélevé chez un individu sain, correspond au diagnostic de l'autisme et/ou de l'autisme lié à la dysbiose intestinale.

10. Méthode selon la revendication 8 ou 9, **caractérisée en ce que** la quantité de l'un au moins des composés définis dans l'une des revendications 1 à 7, présente dans un échantillon biologique prélevé sur un patient, et la quantité de ce(s) même(s) composé(s) présente dans un échantillon biologique prélevé chez un individu sain, est mesurée par chromatographie, par spectrophotométrie, par une méthode immunologique, notamment par un test ELISA, ou immuno-néphélémétrique et préférentiellement par chromatographie en phase gazeuse couplée à une spectrométrie de masse (GC/MS).

11. Méthode selon l'une quelconque des revendications 8 à 10, **caractérisée en ce que** la détermination d'une augmentation de la quantité dudit composé de formule : 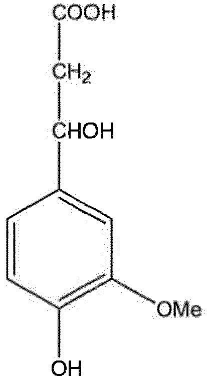 présente dans un échantillon biologique prélevé sur un patient, par rapport à la quantité de ce même composé présente dans un échantillon biologique prélevé chez un individu sain, correspond au diagnostic de l'autisme, de retards de développement ou de l'autisme lié à une dysbiose intestinale.

12. Méthode de diagnostic *in vitro* ou *ex vivo* selon l'une quelconque des revendications 8 à 11, **caractérisée en ce que** l'échantillon biologique est choisi parmi l'urine, le sang, la salive, le liquide cérébrospinal ou les selles.

13. Méthode selon l'une quelconque des revendications 8 à 12, **caractérisée en ce que** la quantité de l'un au moins des composés définis dans l'une des revendications 1 à 7, notamment la quantité du composé acide 3-méthoxy-4-hydroxyphényl-3-hydroxy-propionique, présente dans un échantillon d'urine prélevé sur un patient, est supérieure à environ 20 microgrammes par milligramme de créatinine et notamment de 20 à 180 microgrammes par milligramme de créatinine.

14. Utilisation d'anticorps monoclonaux ou polyclonaux dirigés contre l'un au moins des composés définis dans une quelconque des revendications 1 à 7, notamment dirigés contre l'acide 3-méthoxy-4-hydroxyphényl-3-hydroxy-propionique, en tant que réactifs susceptibles de détecter la présence de l'un au moins desdits composés dans un échantillon biologique, comme marqueur physiologique pour la mise en oeuvre d'une méthode de diagnostic *in vitro* ou *ex vivo* de désordres psychiatriques appartenant au groupe constitué de l'autisme, des retards de développement et de l'autisme lié à la dysbiose intestinale.

## Patentansprüche

1. Verwendung von mindestens einer der Verbindungen mit der folgenden Formel (I): 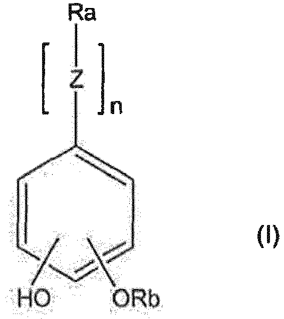 worin:
* n = 0 oder 1
* Ra COOH darstellt
* Rb eine Alkylgruppe mit 1 bis 3 Kohlenstoffatomen, vorzugsweise eine -CH₃-Gruppe darstellt,
* Z -CHOH-CH₂-, -CH₂-CH₂-, -CO-NH-CH₂- darstellt,
* wobei die Verbindungen gegebenenfalls in Salzform oder bei Vorhandensein mindestens eines asymmetrischen Kohlenstoffs in der Form von isolierten Isomeren oder als racemisches Gemisch vorliegen,
* wobei die Verbindungen verschieden von Vanillylmilchsäure und Homovanillinsäure sind,
als physiologischer Marker für die Durchführung eines Verfahrens zur In-vitro- oder Ex-vivo-Diagnose von psychiatrischen Störungen aus der Gruppe bestehend aus Autismus, Entwicklungsverzögerungen und mit Darmdysbiose verbundenem Autismus.

2. Verwendung nach Anspruch 1 von mindestens einer der Verbindungen mit der folgenden Formel (I'): 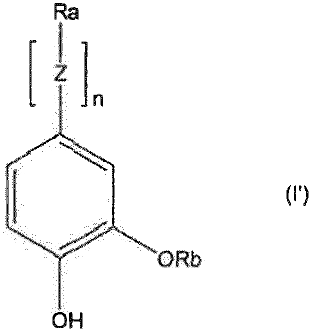 worin:
* n = 0 oder 1
* Ra COOH darstellt
* Rb eine Alkylgruppe mit 1 bis 3 Kohlenstoffatomen, vorzugsweise eine -CH₃-Gruppe darstellt,
* Z -CHOH-CH₂-, -CH₂-CH₂-, -CO-NH-CH₂- darstellt,
* wobei die Verbindungen gegebenenfalls in Salzform oder bei Vorhandensein von mindestens einem asymmetrischen Kohlenstoff in Form von isolierten Isomeren oder als racemisches Gemisch vorliegen,
* wobei die Verbindungen verschieden von Vanillylmilchsäure und Homovanillinsäure sind,
als physiologischer Marker zur Durchführung eines Verfahrens zur In-vitro- oder Ex-vivo-Diagnose von psychiatrischen Störungen aus der Gruppe bestehend aus Autismus, Entwicklungsverzögerungen und mit Darmdysbiose verbundenem Autismus.

3. Verwendung nach einem der Ansprüche 1 oder 2 von mindestens einer der Verbindungen mit der folgenden Formel (II): 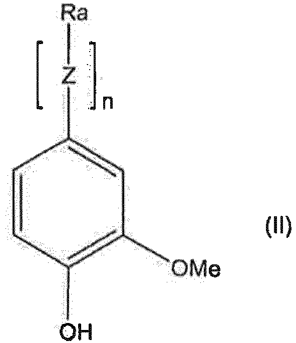 worin n, Ra und Z wie in den Ansprüchen 1 bis 2 definiert sind.

4. Verwendung nach einem der Ansprüche 1 bis 3 von mindestens einer der Verbindungen der Formel (I) oder (II), worin:
* n = 1,
* Ra -COOH darstellt,
* Rb -CH₃- darstellt,
* Z -CHOH-CH₂- darstellt,
* wobei die Verbindungen verschieden von Vanillylmilchsäure sind.

5. Verwendung nach Anspruch 3, **dadurch gekennzeichnet, dass** die Verbindung der Formel (II) aus einer der folgenden Verbindungen ausgewählt ist:

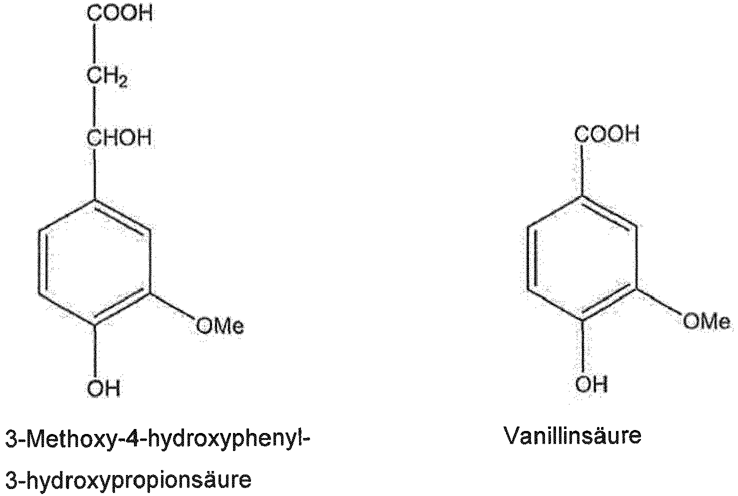

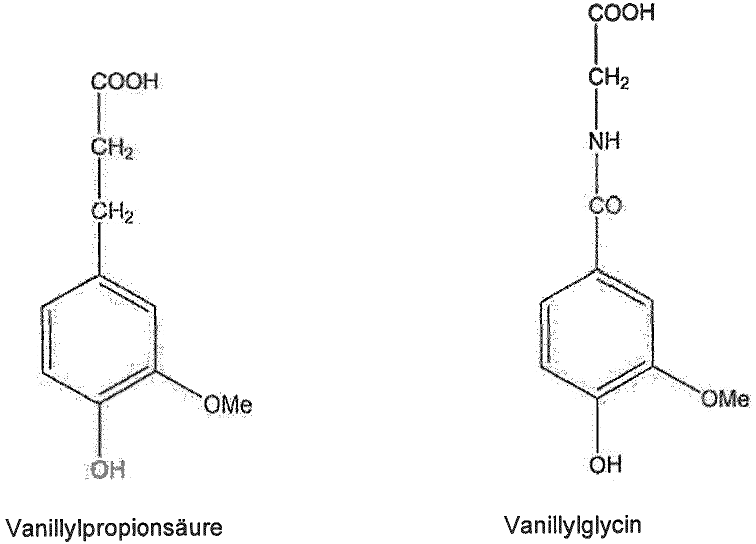

6. Verwendung nach Anspruch 3, **dadurch gekennzeichnet, dass** die Verbindung der folgenden Formel entspricht:

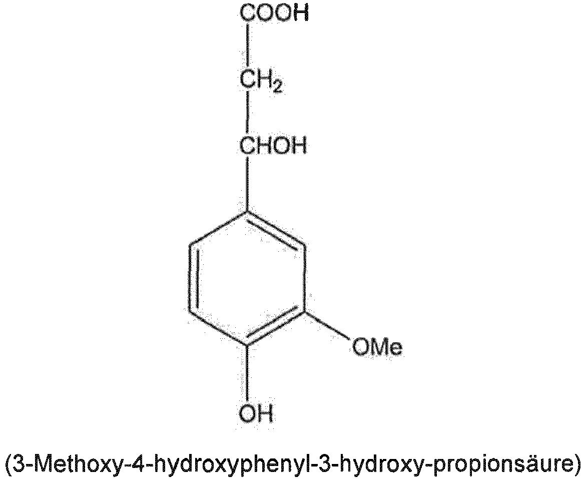

7. Verwendung nach Anspruch 3, **dadurch gekennzeichnet, dass** die Verbindung der folgenden Formel entspricht:

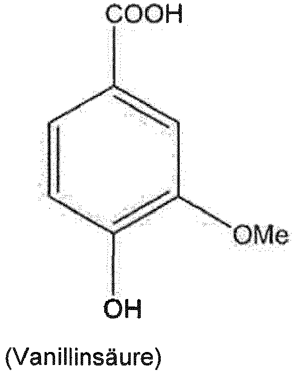

8. Verfahren zur In-vitro- oder Ex-vivo-Diagnose von psychiatrischen Störungen aus der Gruppe bestehend aus Autismus, Entwicklungsverzögerungen und mit Darmdysbiose verbundenem Autismus, **dadurch gekennzeichnet, dass** es einen Schritt der Bestimmung einer Abweichung der Menge von mindestens einer der Verbindungen, die in einem der Ansprüche 1 bis 7 definiert sind, die in einer von einem Patienten entnommenen biologischen Probe vorhanden ist, bezogen auf die Menge dieser gleichen Verbindung/-Verbindungen, die in einer von einem gesunden Individuum entnommenen biologischen Probe vorhanden ist, umfasst.

9. Verfahren nach Anspruch 8, **dadurch gekennzeichnet, dass** die Bestimmung einer Zunahme der Menge von mindestens einer der Verbindungen, die in einem der Ansprüche 1 bis 7 definiert sind, in einer von einem Patienten entnommenen biologischen Probe vorhanden ist, bezogen auf die Menge dieser gleichen Verbindung/Verbindungen, die in einer von einem gesunden Individuum entnommen biologischen Probe vorhanden ist, durch ein geeignetes Verfahren der Diagnose von Autismus und/oder mit Darmdysbiose verbundenem Autismus entspricht.

10. Verfahren nach Anspruch 8 oder 9, **dadurch gekennzeichnet, dass** die Menge von mindestens einer der Verbindungen, die in den Ansprüchen 1 bis 7 definiert sind, die in einer von einem Patienten entnommenen biologischen Probe vorhanden ist, und die Menge dieser gleichen Verbindung/-Verbindungen, die in einer von einem gesunden Individuum entnommenen biologischen Probe vorhanden ist, durch Chromatographie, durch Spektrophotometrie, durch ein immunologisches Verfahren, insbesondere durch einen ELISA- oder immuno-nephelometrischen Test, und vorzugsweise durch mit Massenspektrometrie gekoppelte Gasphasenchromatographie (GC/MS) gemessen wird.

11. Verfahren nach einem der Ansprüche 8 bis 10, **dadurch gekennzeichnet, dass** die Bestimmung einer Zunahme der Menge der Verbindung mit der Formel: 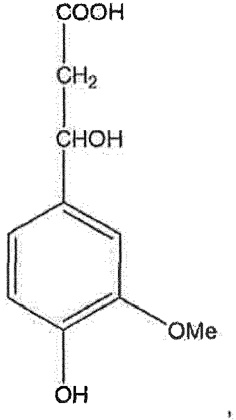 die in einer von einem Patienten entnommenen biologischen Probe vorhanden ist, bezogen auf die Menge dieser gleichen Verbindung, die in einer von einem gesunden Individuum entnommenen biologischen Probe vorhanden ist, der Diagnose von Autismus, Entwicklungsverzögerungen oder mit Darmdysbiose verbundenem Autismus entspricht.

12. Verfahren zur In-vitro- oder Ex-vivo-Diagnose nach einem der Ansprüche 8 bis 11, **dadurch gekennzeichnet, dass** die biologische Probe aus Urin, Blut, Speichel, Liquor cerebrospinalis oder Stuhl ausgewählt wird.

13. Verfahren nach einem der Ansprüche 8 bis 12, **dadurch gekennzeichnet, dass** die Menge von mindestens einer der Verbindungen, die in einem der Ansprüche 1 bis 7 definiert sind, insbesondere die Menge der Verbindung 3-Methoxy-4 hydroxyphenyl-3-hydroxypropionsäure, die in einer von einem Patienten entnommenen Urinprobe vorhanden ist, größer als etwa 20 Mikrogramm pro Milligramm Kreatinin und insbesondere von 20 bis 180 Mikrogramm pro Milligramm Kreatinin ist.

14. Verwendung von monoklonalen oder polyklonalen Antikörpern, die gegen mindestens eine der Verbindungen, die in einem der Ansprüche 1 bis 7 definiert sind, gerichtet sind, und insbesondere gegen 3-Methoxy-4-hydroxyphenyl-3-hydroxy-propionsäure gerichtet sind, als Reagenzien, die zum Detektieren des Vorhandenseins von zumindest einer dieser Verbindungen in einer biologischen Probe als physiologischer Marker für die Durchführung eines Verfahrens zur In-vitro- oder Ex-vivo-Diagnose von psychiatrischen Störungen aus der Gruppe bestehend aus Autismus, Entwicklungsverzögerungen und mit Darmdysbiose verbundenem Autismus geeignet sind.

## Claims

1. Use of at least one compound of the following formula (I): 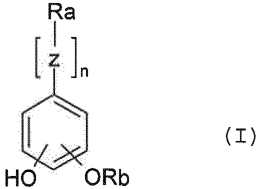 In which,
• n = 0 or 1,
• Ra represents -COOH
• Rb represents an alkyl group having 1 to 3 carbon atoms, preferably a -CH₃ group,
• Z represents -CHOH-CH₂-, -CH₂-CH₂-, -CO-NH-CH₂-,
• wherein said compound may be in the form of salts or, when at least one asymmetric carbon atom is present, in the form of isolated isomers or as a racemic mixture,
• wherein said compound differs from vanillyllactic acid and homovanillic acid,
as a physiological marker for carrying out a method for the *in vitro* or *ex vivo* diagnosis of psychiatric disorders belonging to the group consisting of autism, development retardation and autism linked to intestinal dysbioses.

2. Use according to claim 1 of at least one of the compounds of the following formula (I'): 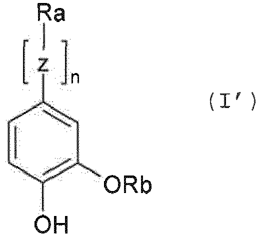 In which,
• n = 0 or 1,
• Ra represents COOH,
• Rb represents an alkyl group having 1 to 3 carbon atoms, preferably a -CH₃ group
• Z represents -CHOH-CH₂-, -CH₂-CH₂-, -CO-NH-CH₂-,
• wherein said compound may be in the form of salts or, when at least one asymmetric carbon atom is present, in the form of isolated isomers or as a racemic mixture,
• wherein said compounds differs from vanillyllactic acid and homovanilic acid,
as a physiological marker for carrying out a method for the *in vitro* or *ex vivo* diagnosis of psychiatric disorders belonging to the group consisting of autism, development retardation and autism linked to intestinal dysbioses.

3. Use according to one of claims 1 or 2 of at least one compound of the following formula (II): 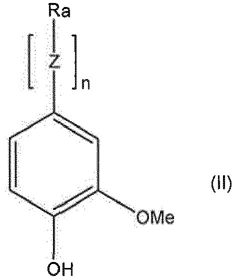
• in which n, Ra, and Z are as defined in claims 1 to 2.

4. Use according to any one of claims 1 to 3, of at least one compound of formula (I) or (II), in which:
• n = 1,
• Ra represents -COOH,
• Rb represents -CH₃,
• Z represents -CHOH-CH₂-,
wherein said compound differs from vanillylactic acid.

5. Use according to claim 3, wherein said compound of formula (II) is selected from the following compounds :

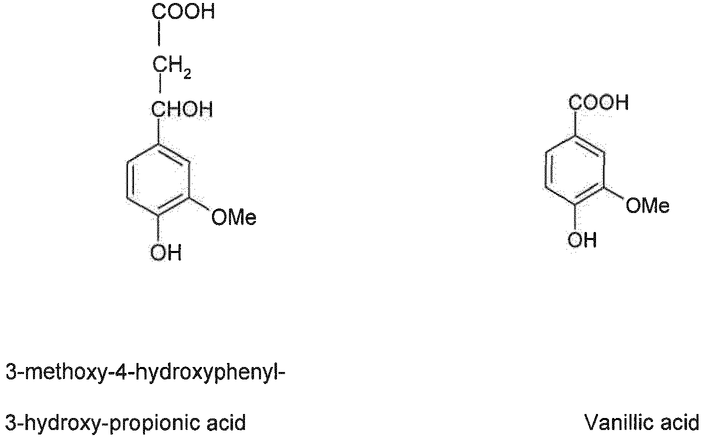

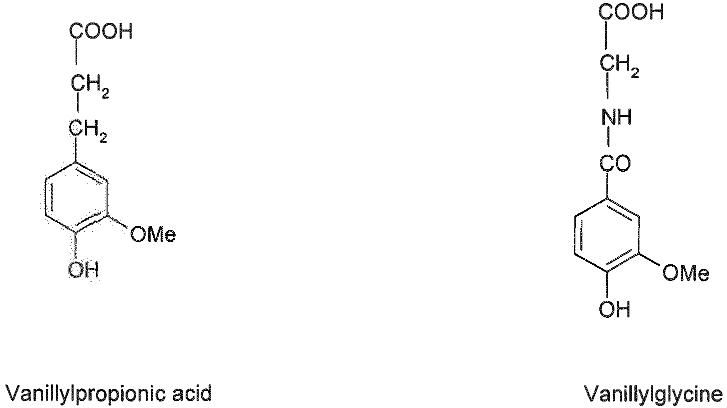

6. Use according to claim 3, **characterized in that** said compound corresponds to the following formula:

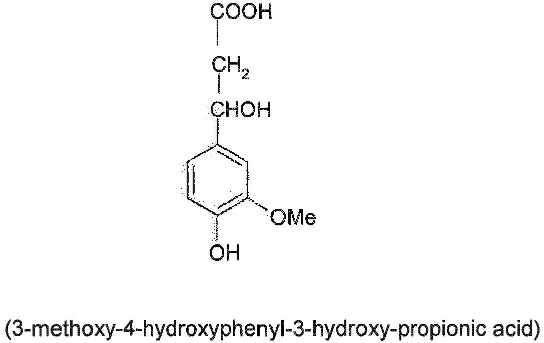

7. Use according to claim 3, **characterized in that** said compound corresponds to the following formula:

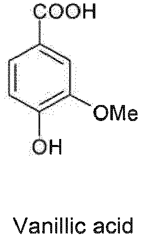

8. A method for the *in vitro* or *ex vivo* diagnosis of psychiatric disorders belonging to the group consisting of autism, development retardation and autism linked to intestinal dysbiose, **characterized in that** said method comprises a step for the determination of a variation in an amount of at least one of the compounds as defined in any one of claims 1 to 7, as present in a biological sample which is taken from a patient, in relation to the amount of this (these) same compound(s) as present in a biological sample which is taken from a healthy individual.

9. A method according to claim 8, **characterized in that** the determination by an appropriate method of an increase in an amount of at least one of the compounds as defined in any one of claims 1 to 7, as present in a biological sample which is taken from a patient, in relation to the amount of this (these) same compound(s) as present in a biological sample which is taken from a healthy individual, corresponds to the diagnosis of autism and/or autism linked to intestinal dysbioses.

10. A method according to claims 8 or 9, **characterized in that** the amount of at least one of the compounds as defined in any one of claims 1 to 7, as present in a biological sample which is taken from a patient, and that the amount of this (these) same compound(s) as present in a biological sample which is taken from a healthy individual, is measured by chromatography, by spectrophotometry, by an immunological method, notably by an ELISA assay, or by an immunonephelemetric method, and preferentially, by gas chromatography, combined with mass spectrometry (GC/MS).

11. A method according to any one of claims 8 to 10, **characterized in that** the determination of an increase in the amount of said compound of the formula: 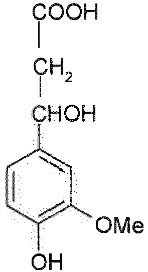 as present in a biological sample which is taken from a patient, in relation to the amount of the same compound as present in a biological sample taken which is taken from a healthy individual, corresponds to the diagnosis of autism, development retardation or autism linked to intestinal dysbiose.

12. A method for the *in vitro* or *ex vivo* diagnosis according to any one of claims 8 to 11, **characterized in that** the biological sample is chosen among urine, blood, saliva, the cerebrospinal fluid or stool.

13. A method according to any one of claims 8 to 12, **characterized in that** the amount of at least one of the compounds as defined in any one of claims 1 to 7, in particular that of 3-methoxy-4-hydroxyphenyl-3-hydroxy-propionic acid, as present in the urine sample which is taken from a patient is superior or equal to 20 micrograms per milligram of creatinine, and notably from 20 to 180 micrograms per milligram of creatinine.

14. Use of monoclonal or polyclonal antibodies against at least one of the compounds as defined in any one of claims 1 to 7, in particular against 3-methoxy-4-hydroxyphenyl-3-hydroxy-propionic acid, as reactants which are able of detecting the presence of at least one of sais compounds in a biological sample, as a physiological marker in carrying out a method for the *in vitro* or ex *vivo* diagnosis of psychiatric disorders belonging to the group consisting of autism, development retardation and autism linked to intestinal dysbiose.
